# EUROPÄISCHE PATENTSCHRIFT

(11) **EP 0 640 603 B1**
(45) Veröffentlichungstag und Bekanntmachung des Hinweises auf die Patenterteilung: **05.02.2003**
(21) Anmeldenummer: 94810430.2
(22) Anmeldetag: 20.07.1994
(51) Int. Cl.: C07D 487/04, C09B 57/04, C08K 5/34

(54) **Neue feinteilige hochtransparente Diketopyrrolopyrrolpigmente**
In fine particles high transparent diketopyrrolopyrrol pigments
Dicétopyrrolopyrrolpigments à haute transparence et en particules fines

(30) Priorität: 29.07.1993 CH 229793
(43) Veröffentlichungstag der Anmeldung: 01.03.1995
(73) Patentinhaber: Ciba Specialty Chemicals Holding Inc., 4057 Basel (CH)
(72) Erfinder: Wallquist, Olof, Dr., CH-1723 Marly (CH); Wooden, Gary, Dr., CH-1716 Oberschrot (CH); Eichenberger, Thomas, Dr., CH-4056 Basel (CH); Schlöder, Ingo, CH-1753 Matran (CH)

(56) Entgegenhaltungen:
- EP-A- 0 061 426
- EP-A- 0 094 911
- EP-A- 0 181 290
- EP-A- 0 302 018
- EP-A- 0 511 165

## Beschreibung

Die vorliegende Erfindung betrifft neue, durch bestimmte Partikelgrössenverteilung gekennzeichnete, feinteilige Diketopyrrolopyrrolpigmente hoher Reinheit und Transparenz und die Herstellung derselben.

1,4-Diketopyrrolo-[3,4-c]-pyrrolpigmente sind seit einigen Jahren bekannt und werden z.B. in EP-A-0 511 165, US-A-4 415 685 und US-A-4 579 949 beschrieben. Einige davon haben sich als hochwertige Pigmente bewährt.

EP-A-0 511 165 und US-A-4 415 685 offenbaren deckende 1,4-Diketopyrrolo-[3,4-c]-pyrrolpigmente.

In jüngerer Zeit ist die Nachfrage nach hochtransparenten Pigmentformen, insbesondere für die Herstellung von Metalleffektlackierungen, erheblich gestiegen. Es stellte sich deshalb das Problem auch von bereits so hochgepriesenen Pigmenten eine reine hochtransparente Form herzustellen.

Im US-A-4 579 949 wird die Herstellung von 1,4-Diketopyrrolo-[3,4-c]-pyrrolen durch Umsetzung eines Bernsteinsäuredialkylesters mit Nitrilen in Gegenwart einer starken Base und anschliessende Hydrolyse des entstandenen Salzes beschrieben. Es wird ausgeführt, dass die Hydrolyse in Wasser, einem Alkohol mit 1 bis 4 C-Atomen oder bevorzugt einer Säure durchgeführt werden soll und, dass transparentere Pigmentformen entstehen, wenn die Hydrolyse unter 80°C vorgenommen wird (unter Hydrolyse versteht man dabei die Ueberführung der Pigmentalkalisalze in das entsprechende Pigment, d.h. die Protonierung der Pigmentalkalisalze).

Aus US-A-4 659 775 sind Verfahren zur Herstellung spezieller Alkyl-diketopyrrolopyrrole und asymmetrischer Diketopyrrolopyrrole ausgehend von Enamindiestern bzw. Pyrrolinonen bekannt. Aus dieser späteren Publikation geht hervor, dass die Hydrolyse bevorzugt in Wasser durchzuführen ist. Bezüglich transparente Formen, wird auch die Durchführung der Hydrolyse unter 80°C vorgeschlagen. Ähnliches wird im ebenso später erschienenen US-A-4 720 305 berichtet, welches die Herstellung von Diketo-pyrrolopyrrol-Pigmentgemischen aus Bernsteinsäurediestern und zwei verschiedenen Nitrilen betrifft. Auch gemäss dieser Publikation wird die Hydrolyse bevorzugt in Wasser durchgeführt. Zur Herstellung transparenter Formen wird hier jedoch eine nachträgliche Zerkleinerung, z.B. durch wässrige Nassmahlung, empfohlen.

US-A-4 931 566 beschreibt ein Verfahren zur Herstellung besonders reiner Pyrrolo-[3,4-c]-pyrrole, welches dadurch gekennzeichnet ist, dass die Hydrolyse sequentiell in mindestens zwei Schritten durchgeführt wird. Die Hydrolyse erfolgt dabei mit einer anorganischen und/oder organischen Säure, mit Wasser und Alkohol oder mit einer anorganischen oder organischen Säure, Wasser oder/und Alkohol, bevorzugt zwischen 50 und 100°C. Über Transparenz wird nichts erwähnt. Bei den spezifisch erwähnten Produkten handelt es sich durchwegs um deckende Pigmente.

Es ist nun gefunden worden, dass die spezielle Kombination dreier Massnahmen bei der Herstellung, nämlich
- Austragen der Pigmentsalz-Suspension auf Wasser und/oder Alkohol,
- Temperaturen zwischen -20° und 50°C,
- Anwesenheit einer Säure
bestimmte feinteilige Diketopyrrolopyrrolpigmente liefert, die dadurch gekennzeichnet sind, dass mindestens 84 Gew.% der Teilchen einen Stokes-äquivalenten Durchmesser (D 84) von ≤ 0,25 µm aufweisen, und sich durch überraschend, bisher unerreicht hohe Reinheit und Transparenz auszeichnen. Die Teilchengrössenverteilung (D 84) wird nach an sich bekannten Methoden, nach Verdünnung einer Lackanreibung, durch Photosedimentometrie (siehe Beispiel 10) bestimmt.

Die vorliegende Erfindung betrifft demnach 1,4-Diketopyrrolo-[3,4-c]-pyrrole der Formel 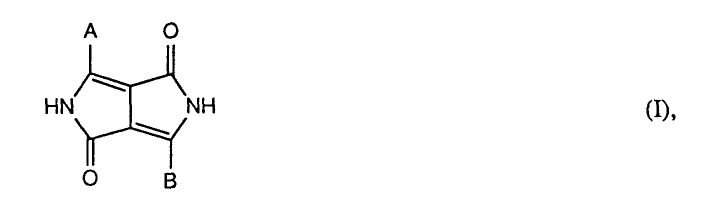 worin A und B unabhängig voneinander einen Rest der Formel 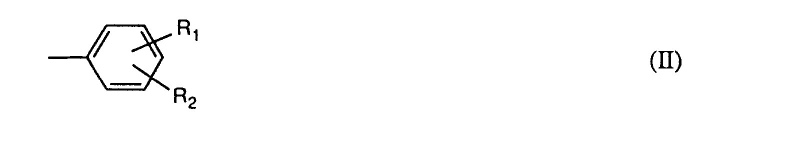 bedeuten, worin R₁ und R₂ unabhängig voneinander Wasserstoff, Halogen, C₁-C₅-Alkyl oder Phenyl sind, die dadurch gekennzeichnet sind, dass mindestens 84 Gew.% der Teilchen einen Stokes-äquivalenten Durchmesser von ≤ 0,25 µm aufweisen, und Gemische davon.

Bevorzugt sind jene 1,4-Diketopyrrolo-[3,4-c]-pyrrole der Formel I, die dadurch gekennzeichnet sind, dass mindestens 84 Gew.% der Teilchen einen Stokes-äquivalenten Durchmesser von ≤ 0,20 µm aufweisen, insbesondere jene worin A und B unabhängig voneinander Reste der Formeln 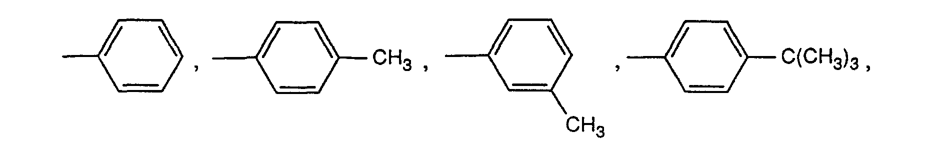 bevorzugt 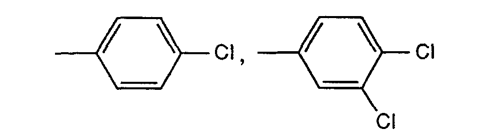 und besonders bevorzugt 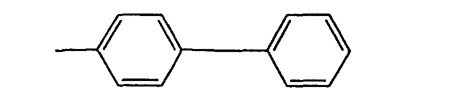 bedeuten.

Das Dₘₐₓ (Durchmesser des Maximums der Häufigkeitsverteilung) ist ≤ 0,15 µm. Aus elektronenmikroskopischen Aufnahmen (Transmission) geht hervor, dass die grösste Menge der Teilchen sogar eine Grösse von etwa 0,01 bis 0,10 µm aufweisen.

Bevorzugt sind die Reste A und B gleich.

Es kann sich aber auch um Gemische, die gegebenenfalls als feste Lösungen oder Mischkristalle vorliegen können, der eben beschriebenen 1,4-Diketopyrrolo-[3,4-c]-pyrrole, insbesondere um das als feste Lösung entstehende Gemisch aus den drei 1,4-Diketopyrrolo-[3,4-c]-pyrrole der Formeln 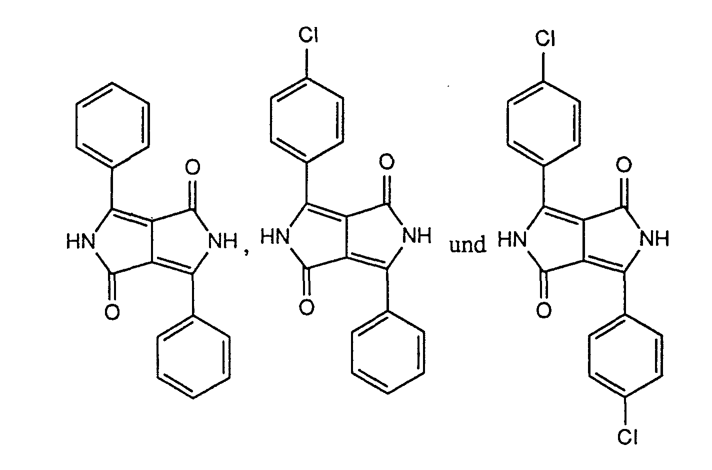 handeln. Das kennzeichnende Röntgenbeugungsdiagramm (bestimmt nach üblichen Methoden mit Hilfe eines Siemens D 500® Röntgen-Difractometers (CuK_{α}-Strahlung)) der soeben beschriebenen festen Lösung ist durch folgende

| Netzebenenabstände (d-Werte in Å) | relative Intensität | doppelte Glanzwinkel (2 Θ) |
|---|---|---|
| 16,1 | 97,1 | 5,5 |
| 7,7 | 22,8 | 11,5 |
| 6,5 | 37,5 | 13,7 |
| 6,0 | 89,8 | 14,7 |
| 5,0 | 20,8 | 17,8 |
| 3,8 | 40,9 | 23,2 |
| 3,7 | 32,4 | 23,7 |
| 3,3 | 100,0 | 26,9 |
| 3,1 | 26,8 | 28,4 |
| 3,0 | 22,4 | 30,0 |

charakterisiert.

Ganz besonders bevorzugt sind die erfindungsgemässen 1,4-Diketopyrrolo-[3,4-c]-pyrrole A, B, C der Formeln 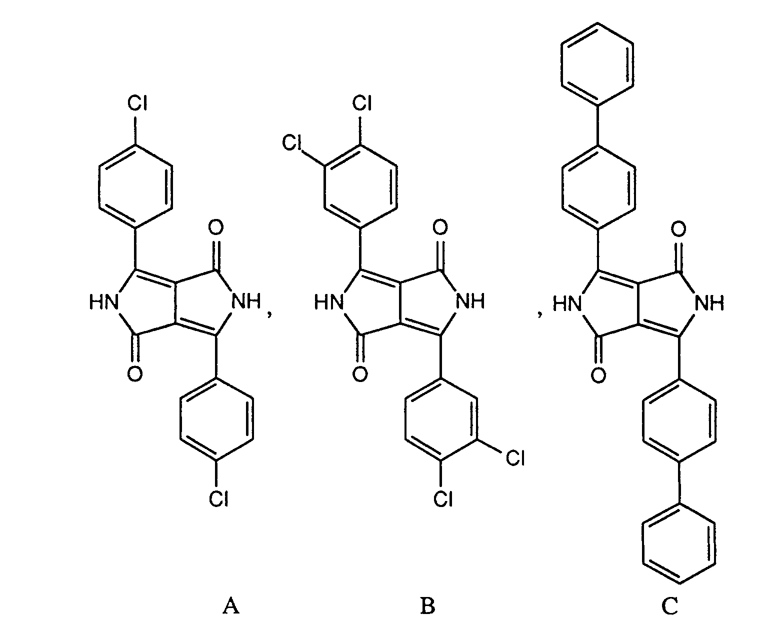 sowie die feste Lösung D aus den drei Pigmenten der Formeln 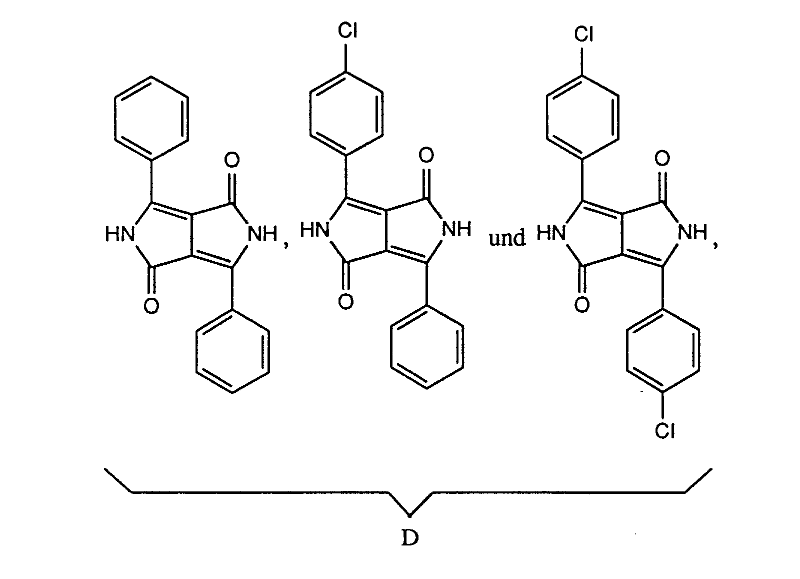 insbesondere jene mit sehr hoher, durch die in nachstehender Tabelle angegebenen Chroma-Mindestwerte gekennzeichneter Sättigung. Es handelt sich dabei um Chroma-Werte C*_{ab} im CIELAB-System, die in einer gemäss DIN 53775, Teil 2, resp. Teil 7 (6.2) hergestellten Vollton-PVC-P-Pressfolie mit einer Pigmentkonzentration von 1 % und 1,0 mm Dicke gemessen werden (vgl. Beispiel 2 sowie Beispiele 4, und 8):

| Pigment | C*_{ab} |
|---|---|
| A | ≥ 42 |
| B | ≥ 42 |
| C | ≥ 14 |
| feste Lösung D | ≥ 36 |

Ein weiterer Gegenstand der vorliegenden Erfindung ist das Verfahren zur Herstellung der eben beschriebenen 1,4-Diketopyrrolo-[3,4-c]-pyrrole der Formel I durch Umsetzung von 1 Mol eines Bemsteinsäure-dicyclohexylesters, -dialkylesters, -monoalkylmonophenylesters oder -diphenylesters, wobei im Bemsteinsäureesterrest Alkyl C₁-C₁₈-Alkyl und Phenyl unsubstituiertes oder durch ein oder zwei Halogenatome, eine oder zwei C₁-C₆-Alkyl- oder C₁-C₆-Alkoxygruppen substituiertes Phenyl bedeuten, mit 2 Mol eines Gemisches von Nitrilen der Formeln

A-CN (III) und B-CN (IV),

worin A und B die oben angegebene Bedeutung haben, wobei im Nitrilgemisch ACN und BCN im Molverhältnis 100:0 bis 50:50 zueinander stehen,
in einem inerten organischen Lösungsmittel in Gegenwart eines Alkalimetalls oder eines Alkali-alkoholates als starke Base bei erhöhter Temperatur zu einem Pigmentalkalimetallsalz und anschliessende Freisetzung einer Verbindung der Formel I durch Protonierung des entstandenen Pigementalkalimetallsalzes und nachfolgende Konditionierung, dadurch gekennzeichnet, dass die Pigmentalkalisalz-Suspension in Wasser und/oder einen Alkohol ROH, worin R C₁-C₄-Alkyl ist, bei einer Temperatur zwischen -20 und 50°C in Gegenwart einer Säure ausgetragen wird und 10 Minuten bis 48 Stunden ebenfalls bei einer Temperatur zwischen -20°C und 50°C, bevorzugt -10 bis 40°C, behandelt wird, mit der Massgabe, dass die Hydrolyse nicht sequentiell in mindestens zwei Schritten ausgeführt wird.

C₁-C₆-Alkyl steht z.B. für Methyl, Ethyl, n-Propyl, Isopropyl, n-Butyl, tert.-Butyl, Amyl, Hexyl und C₁-C₁₈-Alkyl zusätzlich beispielsweise für Heptyl, 2,2-Dimethylhexyl, Octyl, Decyl, Dodecyl, Tetradecyl oder Octadecyl. C₁-C₆-Alkoxy bedeutet z.B. Methoxy, Ethoxy, n-Propoxy, Isopropoxy, Butoxy oder Hexyloxy.

Die Säure kann entweder vor, zusammen mit oder nach der Pigmentsalz-Suspension zugegeben werden, vorzugsweise vor oder zusammen mit der Pigmentsalz-Suspension.

Es kann von Vorteil sein, einen Puffer während der Protonierung zu verwenden, wie beispielsweise einen Phosphat-, Acetat-, Citronensäure- oder Triethanolamin-Puffer.

R bedeutet als C₁-C₄-Alkyl z.B. Methyl, Ethyl, Propyl, Isopropyl, n-Butyl, tert.-Butyl. R ist bevorzugt Methyl oder Ethyl.

Bedeuten R₁ und R₂ C₁-C₅-Alkyl, so handelt es sich z.B. um Methyl, Ethyl, Propyl, Isopropyl, n-Butyl, tert.-Butyl, n-Pentyl und tert.-Amyl.

R₁ und R₂ bedeuten als Halogen insbesondere Brom und bevorzugt Chlor.

Bevorzugt wird die Pigmentalkalisalz-Suspension in ein Wasser/Alkohol-Gemisch ausgetragen, wobei es sich zweckmässig um ein Gemisch von Wasser und Alkohol im Verhältnis 80-20:20-80, bevorzugt 70-30:30-70 und insbesondere 65-35:35-65 Vol% handelt.

Bei den als Protonierungsmittel eingesetzten Säuren handelt es sich z.B. um anorganische Säuren, wie z.B. Salzsäure, Phosphorsäure und insbesondere Schwefelsäure oder um aliphatische oder aromatische Carbon- oder Sulfonsäuren, wie z.B. Ameisensäure, Essigsäure, Propionsäure, Buttersäure, Hexansäure, Oxalsäure, Benzoesäure, Phenylessigsäure, Benzolsulfonsäure oder p-Toluolsulfonsäure oder um Gemische der erwähnten Säuren. Bevorzugte organische Säuren sind Essigsäure und Ameisensäure.

Protonierung und Konditionierung erfolgen bevorzugt bei einer Temperatur zwischen -10° und 30°C während 1 bis 8 Stunden.

Für die Reste A und B gelten die oben bereits angegebenen Bedeutungen und Bevorzugungen.

Bei den zu verwendenden Bernsteinsäuredialkyl- oder -diphenylestern kann es sich um symmetrische oder asymmetrische Diester handeln. Bevorzugt verwendet man aber symmetrische Bernsteinsäurediester, insbesondere symmetrische Bernsteinsäuredialkylester. Liegt ein Bernsteinsäurediphenyl- oder -monophenylmonoalkylester vor, so kann Phenyl beispielsweise unsubstituiertes oder durch ein oder zwei Halogenatome, wie Chlor, C₁-C₆-Alkylgruppen, wie Methyl, Ethyl, Isopropyl oder tert.-Butyl, oder C₁-C₆-Alkoxygruppen, wie Methoxy oder Ethoxy, substituiertes Phenyl bedeuten. Phenyl bedeutet bevorzugt unsubstituiertes Phenyl. Handelt es sich um einen Bernsteinsäuredialkyl- oder -monoalkylmonophenylester, so kann Alkyl unverzweigt oder verzweigt sein, bevorzugt verzweigt, und vorzugsweise 1 bis 12, insbesondere 1 bis 8 und besonders bevorzugt 1 bis 5 C-Atome enthalten. Verzweigtes Alkyl ist bevorzugt sek.- oder tert.-Alkyl, wie z.B. Isopropyl, sek.-Butyl, tert.-Butyl und tert.-Amyl. Ganz bevorzugt verwendet man symmetrische verzweigte Bernsteinsäuredialkylester, worin jeder Alkylrest im Bernsteinsäureesterrest 3 bis 5 C-Atome aufweist.

Beispiele für Bernsteinsäurediester sind Bernsteinsäuredimethylester, -diethylester, -dipropylester, -dibutylester, -dipentylester, -dihexylester, -diheptylester, -dioctylester, -diisopropylester, -di-sec.-butylester, -di-tert.-butylester, -di-tert.-butylester, -di-[1,1-dimethylbutyl]-ester, -di-[1,1,3,3-tetramethylbutyl]-ester, -di-[1,1-dimethylpentyl]-ester, -di-[1-methyl-1-ethyl-butyl]-ester, -di-[1,1-diethylpropyl]-ester, -diphenylester, -di-[4-methylphenyl]-ester, -di-[2-methylphenyl]-ester, -di-[4-chlorphenyl]-ester, -di-[2,4-dichlorphenyl]-ester und -monoethyl-monophenylester.

Die oben aufgeführten Bernsteinsäurediester und die Nitrile der Formel III bzw. IV sind bekannte Verbindungen und können nach bekannten Verfahren hergestellt werden.

Man führt die Umsetzung des Bernsteinsäurediesters mit dem Nitril der Formel III oder IV bzw. einem Gemisch davon in einem organischen Lösungsmittel durch. Als Lösungsmittel eignen sich beispielsweise primäre, sekundäre oder tertiäre Alkohole mit 1 bis 10 C-Atomen, wie Methanol, Ethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Ethyl-3-pentanol und 2,4,4-Trimethyl-2-pentanol, Glykole, wie Ethylenglykol oder Diethylenglykol, ferner Ether, wie Tetrahydrofuran oder Dioxan, oder Glykolether, wie Ethylenglykol-mono- oder -di-methylether, Ethylenglykol-mono- oder -di-ethylether, Diethylenglykol-monomethylether oder Diethylenglykolmonoethylether, ferner dipolar-aprotische Lösungsmittel, wie Acetonitril, Benzonitril, Dimethylformamid, N,N-Dimethylacetamid, Nitrobenzol und N-Methylpyrrolidon, aliphatische oder aromatische Kohlenwasserstoffe, wie Benzol oder durch Alkyl, Alkoxy oder Halogen substituiertes Benzol, wie Toluol, Xylole, Anisol oder Chlorbenzol, oder aromatische N-Heterocyclen, wie Pyridin, Picolin oder Chinolin. Zudem ist es auch möglich, das umzusetzende Nitril der Formel III bzw. IV gleichzeitig als Lösungsmittel im Ueberschuss zu verwenden, falls es im Temperaturbereich flüssig ist, in dem die Umsetzung erfolgt. Die oben genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Im erfindungsgemässen Verfahren verwendet man als Lösungsmittel bevorzugt einen Alkohol, insbesondere einen sekundären oder tertiären Alkohol. Bevorzugte tertiäre Alkohole sind tert.-Butanol und tert.-Amylalkohol. Dabei sind auch Gemische davon, oder Gemische dieser bevorzugten Lösungsmittel mit aromatischen Kohlenwasserstoffen, wie Toluol oder Xylole, oder mit durch Halogen substituierten Benzolen, wie Chlorbenzol oder o-Dichlorbenzol, von grossem Interesse.

Anmeldungsgemäss geeignete starke Basen sind Alkalimetalle, wie Lithium, Natrium und Kalium, und Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, -n-butylat, -sek.butylat, -tert.- butylat, -2-methyl-2-butylat, -2-methyl-2-pentylat, -3-methyl-3-pentylat und -3-ethyl-3-pentylat. Man kann aber auch ein Gemisch der oben genannten Alkalialkoholate verwenden. Bevorzugt verwendet man Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kaliumisopropylat, -sek.-butylat, -tert.-butylat und -tert.-amylat. Dabei können die Alkalialkoholate auch in situ hergestellt werden, indem man den entsprechenden Alkohol mit dem Alkalimetall umsetzt.

Im erfindungsgemässen Verfahren kann man die starke Base beispielsweise in einer Menge von 0,1 bis 10 Mol, bevorzugt 1,9 bis 4,0 Mol, bezogen auf 1 Mol des Bernsteinsäurediesters, einsetzen. Obwohl grundsätzlich stöchiometrische Mengen an Base genügen, beeinflusst in vielen Fällen ein Basenüberschuss die Ausbeute günstig.

Die Umsetzung kann beispielsweise bei einer Temperatur von 60 bis 140°C, vorzugsweise aber von 80 bis 120°C, durchgeführt werden.

Zur Umsetzung des Bernsteinsäurediesters mit dem bzw. den Nitrilen der Formeln III und IV ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zuzugeben. Eine bevorzugte Ausführungsform, die sich in der Regel besonders günstig auf die Ausbeute auswirkt, besteht darin, dass man das umzusetzende Nitril zusammen mit der starken Base vorlegt und den Bernsteinsäurediester im Bereiche der Reaktionstemperatur zudosiert. Eine weitere Möglichkeit besteht darin, dass man den Bernsteinsäurediester und das umzusetzende Nitril gleichzeitig zur vorgelegten Base zudosiert. Es ist durchaus möglich, das erfindungsgemässe Verfahren nicht nur batchweise, sondern auch kontinuierlich durchzuführen.

Insbesondere bei Bernsteinsäurediestern mit niederen Alkylresten und bei Alkoholaten, die sich aus niederen Alkoholen ableiten, wie z.B. Methanol, Ethanol, n-Propanol, Isopropanol oder tert.-Butanol, kann es sich als vorteilhaft erweisen, den bei der Umsetzung entstehenden niederen Alkohol laufend aus dem Reaktionsmedium zu entfernen, um höhere Ausbeuten zu erzielen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Zur Protonierung der erhaltenen Pigmentsalze kann man entweder das Pigmentalkalisalz zum erfindungsgemässen, aus Wasser und/oder Alkohol und der Säure bestehenden Protonierungsmittel oder das Pigmentalkalisalz und die Säure gleichzeitig zum Wasser und/oder Alkohol, oder die Säure erst nach der Zugabe der Pigmentalkalisalz-Suspension zum Wasser und/oder Alkohol zugeben. Das Wasser und/oder der Alkohol kann in beliebigen Mischungsverhältnissen zwischen 5 und 20 Gew.Teilen auf 1 Teil des entstandenen Pigmentalkalisalzes eingesetzt werden. Die Säure wird zweckmässig, je nach Temperatur und Ausgangsmaterial, in einer Menge von 0,5 bis 3, bevorzugt 1 bis 2 Aequivalenten, bezogen auf die Base eingesetzt, vorzugsweise in genügender Menge, um den pH <10 am Ende der Protonierung zu erreichen.

Bei der Herstellung der obenerwähnten 1,4-Diketopyrrolo-[3,4-c]-pyrrole der Formeln 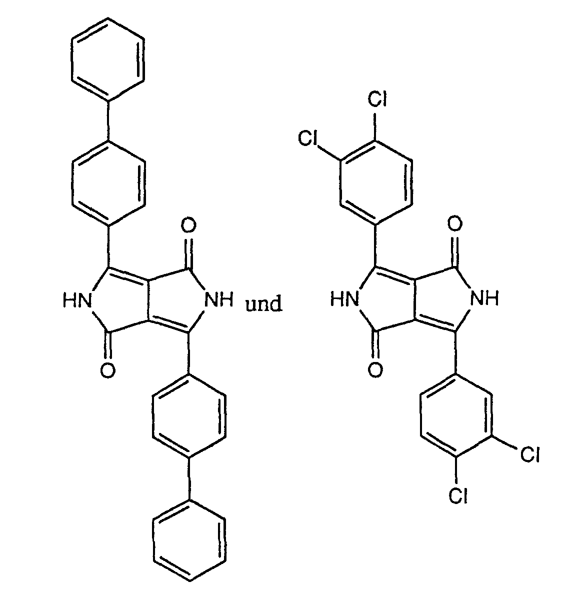 können sogar auch ohne Zusatz von Säure gute Resultate erhalten werden.

Nach der Protonierung fallen die Verbindungen der Formel I aus und können durch an sich bekannte Trennverfahren, wie Abfiltrieren, isoliert werden.

Die Verbindungen der Formel I können als Pigmente für hochmolekulare organische Materialien verwendet werden. Dabei lassen sich die Pigmente meistens direkt in der Pigmentform einsetzen, wie sie nach dem erfindungsgemässen Verfahren anfallen. Ihre Kristallmorphologie kann je nach Verwendungszweck, und sofern notwendig, durch eine der zahlreichen üblichen nachträglichen Behandlungen noch optimiert werden.

Je nach Verwendungszweck kann es vorteilhaft sein, Gemische von Verbindungen der Formel I herzustellen. Dies lässt sich beispielsweise dadurch erreichen, dass man unabhängig voneinander hergestellte, verschiedene Reaktionslösungen vor der Protonierung mischt, sie zusammen protoniert und dann das erhaltene Produkt isoliert oder auch dadurch, dass man bei der Herstellung nicht ein sondern zwei verschiedene Nitrile einsetzt. In vielen Fällen entstehen dabei feste Lösungen oder Mischkristalle.

Hochmolekulare organische Materialien, die mit den Verbindungen der Formel I gefärbt bzw. pigmentiert werden können, sind z.B. Celluloseether und -ester, wie Ethylcellulose, Nitrocellulose, Celluloseacetat und Cellulosebutyrat, natürliche Harze und Kunstharze, wie Polymerisationsharze oder Kondensationsharze, z.B. Aminoplaste, insbesondere Harnstoff- und Melamin-Formaldehydharze, Alkydharze, Phenoplaste, Polycarbonate, Polyolefine, wie Polyethylen und Polypropylen, Polystyrol, Polyvinylchlorid, Polyacrylnitril, Polyacrylsäureester, Polyamide, Polyurethane, Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die Verbindungen der Formel I als Toner oder in Form von Präparaten einzusetzen. Bezogen auf das zu pigmentierende hochmolekulare organische Material kann man die Verbindungen der Formel I in einer Menge von 0,01 bis 30 Gew.-%, vorzugsweise 0,1 bis 20 Gew.-%, einsetzen.

Je nach Konditionierverfahren oder Applikationszweck kann es von Vorteil sein, dem Pigment gewisse Mengen an texturverbessernden Mitteln vor oder nach dem Konditionierprozess zuzufügen, sofern diese keine negative Wirkung bei der Verwendung der erfindungsgemässen Pigmente (insbesondere in Polyethylen) haben. Als solche kommen insbesondere Fettsäuren mit mindestens 18 C-Atomen, beispielsweise Stearin- oder Behensäure oder deren Amide oder Metallsalze, insbesondere Mg-Salze, sowie Weichmacher, Wachse, Harzsäuren, wie Abietinsäure, Kolophoniumseife, Alkylphenole oder aliphatische Alkohole, wie Stearylalkohol oder aliphatische 1,2-Dihydroxyverbindungen mit 8 bis 22 C-Atomen, wie 1,2-Dodecandiol, ferner modifizierte Kolophoniummaleinatharze oder Fumarsäurekolophoniumharze in Betracht. Die texturverbessernden Mittel werden vorzugsweise in Mengen von 0,1-30 Gew.%, insbesondere 2-15 Gew.%, bezogen auf das Endprodukt, zugesetzt. Die obenerwähnten 1,2-Dihydroxyverbindungen, insbesondere 1,2-Dodecandiol, können auch zur Verbesserung der Filtration der suspendierten Pigmentzusammensetzung eingesetzt werden.

Die erhaltenen Ausfärbungen, beispielsweise in Kunststoffen, Fasern, Lacken oder Drucken, zeichnen sich, ausser durch die ausserordentlich hohe Farbton-Reinheit und Transparenz, durch hohe Farbstärke, gute Dispergierbarkeit, gute Ueberlackier-, Migrations-, Hitze-, Licht- und Wetterbeständigkeit, sowie durch einen guten Glanz, aus.

Die erfindungsgemässen Verbindungen der Formel I zeichnen sich aber, wie bereits erwähnt, ganz besonders durch eine hervorragende Farbton-Reinheit und durch ihre hohe Transparenz aus. Demnach eignen sie sich vorzugsweise zum Färben von Kunststoffen, Druckfarben und wässrigen und/oder lösungsmittelhaltigen Lacken, insbesondere Automobillacken. Ganz besonders bevorzugt ist ihre Verwendung für Metalleffecktlackierungen (Metall oder Mica).

Die nachfolgenden Beispiele dienen zur Erläuterung der Erfindung. Sofern nichts anderes vermerkt, bedeuten Prozente Gewichtsprozente.

Beispiel 1: In einem Sulfierkolben werden unter Stickstoff 450 ml tert.-Amylalkohol vorgelegt. Nach der Zugabe von 29,9 g Natrium wird das Gemisch auf 95-102°C aufgewärmt. Das geschmolzene Natrium wird nun unter heftigem Rühren über Nacht bei 100-105°C gehalten. Zur entstandenen Lösung werden dann 33,8 g Benzonitril und 44,8 g 4-Chlorbenzonitril zugegeben. Anschliessend werden innerhalb von 5 Stunden bei 105-110°C 86,0 g Bernsteinsäure-diisopropylester zudosiert, das entstehende Isopropanol gleichzeitig abdestilliert und kontinuierlich mit t-Amylalkohol ersetzt. Das Reaktionsgemisch wird 2 Stunden weiter gerührt, auf Raumtemperatur abgekühlt und noch mit 100 ml tert.-Amylalkohol verdünnt. Anschliessend wird das Reaktionsgemisch auf eine Mischung von 345 ml Wasser, 345 ml Methanol und 70 ml konzentrierte Schwefelsäure bei 20°C zugeführt und dann bei 25°C 4 Stunden gerührt.
Das dunkelrote Gemisch wird filtriert, mit Methanol und Wasser gewaschen und das Pigment bei 80°C im Vakuumtrockenschrank getrocknet. Man erhält 87,8 g eines roten Pulvers.

| Analyse | C | H | N | Cl |
|---|---|---|---|---|
| Berechnet* | 67,0 % | 3,4 % | 8,7 % | 11,0 % |
| Gefunden | 66,7 % | 3,4 % | 8,6 % | 11,2 % |

| | | | | |
|---|---|---|---|---|
| * mit der Annahme gleicher Reaktivität der beiden Nitrile | | | | |

### Partikelgrössenverteilung:

84 Gew.% der Pigmentteilchen weisen einen Stokes-äquivalenten Durchmesser D 84 von ≤ 0,14 µm auf. Das Dₘₐₓ beträgt 0,10 µm.

Beispiel 2: Man verfährt wie in Beispiel 1, verwendet jedoch 25,4 g Na, 315 ml t-Amylalkohol, 28,8 g Benzonitril, 38,1 g 4-Chlorbenzonitril, 72,6 g Bernsteinsäure und 56,4 g konzentrierter Schwefelsäure. Man erhält 76,1 g eines roten Pulvers.

| Analyse | C | H | N | Cl |
|---|---|---|---|---|
| Berechnet* | 67,0 % | 3,4 % | 8,7 % | 11,0 % |
| Gefunden | 66,6 % | 3,4 % | 8,6 % | 11,1 % |

| | | | | |
|---|---|---|---|---|
| * mit der Annahme gleicher Reaktivität der beiden Nitrile | | | | |

### Partikelgrössenverteilung:

84 Gew.% der Pigmentteilchen weisen einen Stokes-äquivalenten Durchmesser D 84 von ≤ 0,11 µm auf. Das Dₘₐₓ beträgt 0,09 µm.

### Sättigung (Chroma):

Mit dem erhaltenen Produkt wird nach der in DIN 53775, Teil 2, resp. Teil 7 (6.2, Walzentemperatur 75°C) festgelegten Methode eine PVC-P-Pressfolie von 1,0 µm Dicke (vgl. Punkt 6.3 von DIN 53775, Teil 2) mit 1 % Pigmentkonzentration hergestellt und der Chroma (C*_{ab})-Wert nach CIELAB bestimmt. Alle Farbmessungen wurden mittels eines Minolta CM-2002® Spectrophotometers (d/8 Geometrie, Messung über Weiss unter Einschluss des Glanzes, Lichtart D 65, Beobachter 10°) ausgeführt.

1 % Pigment in einer Vollton-PVC-P-Pressfolie von 1,0 µm Dicke weist ein Chroma C*_{ab} von 41,6 auf.

Beispiel 3: In einem Sulfierkolben werden unter Stickstoff 170 ml tert.-Amylalkohol vorgelegt. Nach der Zugabe von 11,04 g Natrium wird das Gemisch auf 92-102°C aufgewärmt. Das geschmolzene Natrium wird unter heftigem Rühren über Nacht bei 100-107°C gehalten. Zur entstandenen Lösung werden dann 44,02 g 4-Chlorbenzonitril und 37,2 g Bernsteinsäure-diisopropylester, gelöst in 50 ml tert.-Amylalkohol bei 80°C, innerhalb von 2 Stunden bei 80-98°C zudosiert. Das Reaktionsgemisch wird 3 Stunden bei 80°C weitergerührt und gleichzeitig werden 4,88 g Bernsteinsäure-diisopropylester zugetropft. Anschliessend wird das Reaktionsgemisch auf Raumtemperatur abgekühlt, einer Mischung aus 270 ml Methanol, 200 ml Wasser und 48,1 g konzentrierter Schefelsäure bei 20°C zugegeben und bei 20°C 6 Stunden gerührt. Das rote Gemisch wird filtriert, der Rückstand wird mit Methanol und Wasser gewaschen und bei 80°C im Trockenschrank getrocknet. Man erhält 46,7 g eines roten Pulvers.

| Analyse | C | H | N | Cl |
|---|---|---|---|---|
| Berechnet | 60,50 % | 2,82 % | 7,84 % | 19,85 % |
| Gefunden | 60,36 % | 2,85 % | 7,69 % | 19,61 % |

### Partikelgrössenverteilung:

84 Gew.% der Pigmentteilchen weisen einen Stokes-äquivalenten Durchmesser D 84 von ≤ 0,14 µm auf. Das Dₘₐₓ beträgt 0,11 µm.

Beispiel 4: In einem Sulfierkolben werden unter Stickstoff 170 ml tert.-Amylalkohol vorgelegt. Nach der Zugabe von 11,04 g Natrium wird das Gemisch auf 92-102°C aufgewärmt. Das geschmolzene Natrium wird unter heftigem Rühren über Nacht bei 100-107°C gehalten. Zur entstandenen Lösung werden dann 44,2 g 4-Chlorbenzonitril und 37,2 g Bernsteinsäure-diisopropylester gelöst in 50 ml tert.-Amylalkohol bei 80°C, innerhalb von 2 Stunden bei 80-98°C zugetropft. Das Reaktionsgemisch wird 3 Stunden bei 80°C weiter gerührt und gleichzeitig werden 4,88 g Bernsteinsäure-diisopropylester zugetropft. Anschliessend wird das Reaktionsgemisch auf Raumtemperatur abgekühlt, einer Mischung aus 170 ml Methanol, 320 ml Wasser und 48,1 g konzentrierter Schefelsäure bei 0°C zugegeben und bei 0°C 6 Stunden gerührt. Das rote Gemisch wird filtriert, der Rückstand wird mit Methanol und Wasser gewaschen und bei 80°C im Vakuumtrockenschrank getrocknet. Man erhält 45,5 g eines roten Pulvers.

| Analyse | C | H | N | Cl |
|---|---|---|---|---|
| Berechnet | 60,50 % | 2,82 % | 7,84 % | 19,85 % |
| Gefunden | 60,32 % | 2,90 % | 7,84 % | 19,55 % |

### Partikelgrössenverteilung:

84 Gew.% der Pigmentteilchen weisen einen Stokes-äquivalenten Durchmesser D 84 von ≤ 0,17 µm auf. Das Dₘₐₓ beträgt 0,10 µm.

### Sättigung (Chroma):

1 % Pigment in einer Vollton-PVC-P-Pressfolie von 1,0 mm Dicke weist ein Chroma C*_{ab} von 45,7 auf.

Beispiel 5: In einem Sulfierkolben werden unter Stickstoff 190 ml tert.-Amylalkohol vorgelegt. Nach der Zugabe von 10,6 g Natrium wird das Gemisch auf 92-102°C aufgewärmt. Das geschmolzene Natrium wird unter heftigem Rühren über Nacht bei 100-107°C gehalten. Zur entstandenen Lösung werden dann 41,22 g 4-Biphenylnitril bei 85-90°C zugegeben. Man heizt wieder auf 105-110°C und tropft innerhalb von 6 Stunden 27,9 g Bernsteinsäure-diisopropylester zu. Das Reaktionsgemisch wird 3 Stunden bei 100°C weiter gerührt. Anschliessend wird das Reaktionsgemisch auf Raumtemperatur abgekühlt, einer Mischung aus 160 ml Methanol, 140 ml Wasser und 46,0 g konzentrierter Schefelsäure bei 0°C zugegeben und bei der gleichen Temperatur 5 Stunden gerührt. Das violette Gemisch wird filtriert, der Rückstand wird mit Methanol und Wasser gewaschen und bei 80°C im Vakuumtrockenschrank getrocknet. Man erhält 43,4 g eines dunkelroten Pulvers.

| Analyse | C | H | N |
|---|---|---|---|
| Berechnet | 81,80 % | 4,58 % | 6,36 % |
| Gefunden | 81,17 % | 4,60 % | 6,25 % |

### Partikelgrössenverteilung:

84 Gew.% der Pigmentteilchen weisen einen Stokes-äquivalenten Durchmesser D 84 von ≤ 0,21 µm auf. Das Dₘₐₓ beträgt 0,11 µm.

### Sättigung (Chroma):

1 % Pigment in einer Vollton-PVC-P-Pressfolie von 1,0 mm Dicke weist ein Chroma C*_{ab} von 15,8 auf.

Beispiel 6: In einem Sulfierkolben werden unter Stickstoff 200 ml tert.-Amylalkohol vorgelegt. Nach der Zugabe von 16,14 g Natrium wird das Gemisch auf 92-102°C aufgewärmt. Das geschmolzene Natrium wird unter heftigem Rühren über Nacht bei 100-107°C gehalten. Zur entstandenen Lösung werden dann 41,94 g 3-Tolunitril bei 95-100°C zugegeben. Man heizt wieder auf 105-110°C und tropft innerhalb von 5 Stunden 46,14 g Bernsteinsäure-diisopropylester zu. Das Reaktionsgemisch wird 3 Stunden bei 100°C weiter gerührt. Anschliessend wird das Reaktionsgemisch auf Raumtemperatur abgekühlt, einer Mischung aus 190 ml Methanol, 190 ml Wasser und 71,6 g konzentrierter Schefelsäure bei 0°C zugegeben und bei 0°C 5 Stunden gerührt. Das rote Gemisch wird filtriert, der Rückstand wird mit Methanol und Wasser gewaschen und bei 80°C im Vakuumtrockenschrank getrocknet. Man erhält 40,9 g eines roten Pulvers.

| Analyse | C | H | N |
|---|---|---|---|
| Berechnet | 75,93 % | 5,10 % | 8,85 % |
| Gefunden | 75,82 % | 2,23 % | 8,71 % |

### Partikelgrössenverteilung:

84 Gew.% der Pigmentteilchen weisen einen Stokes-äquivalenten Durchmesser D 84 von ≤ 0,16 µm auf. Das Dₘₐₓ beträgt 0,13 µm.

Beispiel 7: In einem Sulfierkolben werden unter Stickstoff 200 ml tert.-Amylalkohol vorgelegt. Nach der Zugabe von 9,2 g Natrium wird das Gemisch auf 92-102°C aufgewärmt. Das geschmolzene Natrium wird unter heftigem Rühren über Nacht bei 100-107°C gehalten. Zur entstandenen Lösung werden dann 31,24 g 4-Tolunitril bei 85-90°C zugegeben. Man heizt wieder auf 95°C und tropft innerhalb von 6 Stunden 32,36 g Bernsteinsäure-diisopropylester zu. Das Reaktionsgemisch wird 2 Stunden bei 95°C weiter gerührt. Anschliessend wird das Reaktionsgemisch auf Raumtemperatur abgekühlt, einer Mischung von 180 ml Methanol, 180 ml Wasser und 40,8 g konzentrierter Schefelsäure bei 0°C zugegeben, und bei 0°C 5 Stunden gerührt. Das rote Gemisch wird filtriert, der Rückstand wird mit Methanol und Wasser gewaschen und bei 80°C im Vakuumtrockenschrank getrocknet. Man erhält 19,8 g eines roten Pulvers.

| Analyse | C | H | N |
|---|---|---|---|
| Berechnet | 75,93 % | 5,10 % | 8,86 % |
| Gefunden | 75,57 % | 5,11 % | 8,63 % |

### Partikelgrössenverteilung:

84 Gew.% der Pigmentteilchen weisen einen Stokes-äquivalenten Durchmesser D 84 von ≤ 0,18 µm auf. Das Dₘₐₓ beträgt 0,14 µm.

Beispiel 8: In einem Sulfierkolben werden unter Stickstoff 200 ml tert.-Amylalkohol vorgelegt. Nach der Zugabe von 10,3 g Natrium wird das Gemisch auf 92-102°C aufgewärmt. Das geschmolzene Natrium wird unter heftigem Rühren über Nacht bei 100-107°C gehalten. Zur entstandenen Lösung werden dann nach Abkühlung auf 80°C 38,5 g 3,4-Dichlorbenzonitril zugegeben. Bei 80°C werden dann innerhalb von 2¹/₂ Stunden 29,4 g Bernsteinsäure-diisopropylester zugetropft. Nach weiterem Rühren während 2 Stunden bei 80°C wird auf Raumtemperatur abgekühlt. Das Reaktionsgemisch wird danach einer Mischung aus 160 ml Methanol, 160 ml Wasser und 11,4 g konzentrierter Schefelsäure bei 50°C zugegeben und 4 Stunden bei 50°C gerührt. Das rote Gemisch wird filtriert und der Rückstand wird mit Methanol und Wasser gewaschen und bei 80°C im Vakuumtrockenschrank getrocknet. Man erhält 40,6 g eines roten Pulvers.

| Analyse | C | H | N | Cl |
|---|---|---|---|---|
| Berechnet | 50,74 % | 1,89 % | 6,57 % | 33,28 % |
| Gefunden | 49,12 % | 2,18 % | 6,21 % | 29,15 % |

### Partikelgrössenverteilung:

84 Gew.% der Pigmentteilchen weisen einen Stokes-äquivalenten Durchmesser D 84 von ≤ 0,06 µm auf. Das Dₘₐₓ beträgt 0,04 µm.

### Sättigung (Chroma):

1 % Pigment in einer Vollton-PVC-P-Pressfolie von 1,0 mm Dicke weist ein Chroma C*_{ab} von 45,2 auf.

Beispiel 9: In einem Sulfierkolben werden unter Stickstoff 450 ml tert.-Amylalkohol vorgelegt. Nach der Zugabe von 26,5 g Natrium wird das Gemisch auf 92-102°C aufgewärmt. Das geschmolzene Natrium wird unter heftigem Rühren über Nacht bei 100-107°C gehalten. Zur entstandenen Lösung werden dann 111,0 g 4-Biphenylnitril zugegeben. Bei 105-110°C werden 69,8 g Bernsteinsäure-diisopropylester während 4 Stunden zugetropft. Das Reaktionsgemisch wird bei 82°C während 9 Stunden weitergerührt. Anschliessend wird das Reaktionsgemisch auf Raumtemperatur abgekühlt, einer Mischung aus 1100 ml Methanol, 250 ml Wasser und 47,0 g 60 %iger Schwefelsäure bei 26-45°C zugetropft und dann 5 Stunden bei 45°C gerührt. Das Gemisch wird danach filtriert, der Rückstand wird mit Methanol und Wasser gewaschen und bei 80°C im Vakuumtrockenschrank getrocknet. Man erhält 100,6 g eines dunkelroten Pulvers.

| Analyse | C | H | N |
|---|---|---|---|
| Berechnet | 81,80 % | 4,58 % | 6,36 % |
| Gefunden | 80,10 % | 4,57 % | 6,26 % |

### Partikelgrössenverteilung:

84 Gew.% der Pigmentteilchen weisen einen Stokes-äquivalenten Durchmesser D 84 von ≤ 0,13 µm auf. Das Dₘₐₓ beträgt 0,11 µm.

Beispiel 10: 5 g des Pigments aus Beispiel 1, 56,56 g Alkydharz ®ALKYDAL F310 (Bayer), 60 %ig in Xylol, 21,70 g Xylol, 0,94 g Silikonöl (1 %ig in Xylol) und 13,55 g Melaminharz ®CYMEL 327 (Cyanamid), 90 % in Isobutanol werden zusammen in einer Schüttelmaschine (®Skandex-Disperser BA-S 20 in Anlehnung an DIN 53238, Teil 10) bis Stufe 6 (DIN 53238, Teil 24) erreicht wird, jedoch maximal 4 Stunden, dispergiert.

Der so erhaltene Volltonlack kann nach Verdünnung nach üblichen Methoden für die Bestimmung der Teilchengrössenverteilung durch Photosedimentometrie (vgl. Herbst & Hunger, Industrielle Organische Pigmente, VCH 1987, S. 32-34 und 40-43 und K. Brugger, Powder Technology 13, 215-221 (1976)) verwendet werden. Die Teilchengrössenverteilungen werden mit einem Joyce-Loebl Scheibenzentrifugen-Photodensitometer 4 (DCF 4) nach standard Methoden gemessen (vgl. K. Brugger, Powder Technology 13, 215-221 (1976); K. Brugger, Powder Technology 14, 187-188 (1976); F.K. Hansen in: ACS Symp. Ser. 471 (Particle Size Distribution II), 169-183 (1991); R. Finsy et al., in: Advances in Measurement and Control of Colloidal Processes, Int. Symp. Butterworth, London (1991); Instruction manual of the DCF 4, Joyce-Loebl Ltd., Gateshead, UK). 0,5 ml des verdünnten (Verdünnung 1:39 mit einem an den Volltonlack adaptierten organischen Medium) und ultrabeschallten (Tip sonifier Branson B 12, 60 sec, 100 W Ausgangsleistung) Volltonlackes werden auf 15 ml Spinfluid aufgetragen. Das Spinfluid besteht aus einem Dichtegradienten von 20 bis 40 Gew% Tetrachlorethylen in dem Volltonlack-adaptierten Medium bei Raumtemperatur, und wird mit einem Gradienten-Mischer (W. Alex, Dissertation, Universität Karlsruhe (1972)) aufgebaut. Die DCF 4 wird mit 10000 UpM betrieben. Die Extinktion des Weisslichts beruht auf Streuung und/oder Absorption der Teilchen, welche den Lichtstrahl nahe des äusseren Scheibenrandes durchqueren. Das transmittierte Licht wird mit einer Photodiode detektiert und in Abhängigkeit von der Zeit in Intervallen von ca. 0,1 Sek. aufgezeichnet (total Messzeit etwa 40 Min.). Die Zeitwerte werden nach dem Stokes'schen Sedimentationsgesetz in Teilchengrössen, und die Transmissionsdaten unter Verwendung der Mie-Theorie in relative Massen umgerechnet (Wellenlängen-gemittelte Extinktionseffizienz vgl. F.K. Hansen in: ACS Symp. Ser. 471 (Particle Size Distribution II), 169-183 (1991); Light Scattering by Small Particles, H.C. van de Hulst, Wiley, London (1957); H. Reichert, Powder Technology 6, 363-366 (1972)). Vor der Umrechnung werden die Werte jedes Datenpaares über 1 bis 1000 Einzelmessungen gemittelt, um das Signal-Rausch-Verhältnis zu optimieren. die Temperaturabhängigkeit von Dichte und Viskosität des Spinfluids werden bei der Auswertung berücksichtigt. Im vorliegenden Fall weisen mindestens 84 Gew.% der Teilchen einen Stokes-äquivalenten Durchmesser D 84 von ≤0,25 µm auf.

Der erhaltene Volltonlack kann auch mit einem Spiralrakel (100 µm Nassfilm) auf eine Polyethylen-Transparentfolie appliziert werden. Der Lack wird dann 15 Minuten bei Raumtemperatur abdunsten gelassen und anschliessend 30 Minuten bei 115°C eingebrannt.

Beispiel 11: 7,5 g des Pigments aus Beispiel 1, 98,9 g CAB-Lösung bestehend aus

| | |
|---|---|
| 41,0 g | Celluloseacetobutyrat ®CAB 531.1, 20 %ig in Butanol/Xylol 2:1 (Eastman Chem.) |
| 1,5 g | Zirkonium Octoat, |
| 18,5 g | ®SOLVESSO 150* (ESSO), |
| 21,5 g | Butylacetat und |
| 17,5 g | Xylol, |

| | |
|---|---|
| * Aromatische Kohlenwasserstoffe | |

36,5 g Polyesterharz ®DYNAPOL H700 (Dynamit Nobel), 4,6 g Melaminharz MAPRENAL MF650 (Hoechst) und 2,5 g Dispergiermitel ®DISPERBYK 160 (Byk Chemie) werden zusammen während 90 Minuten mit einer Schüttelmaschine dispergiert (Total Lack 150 g; 5 % Pigment).

27,69 g des so erhaltenen Volltonlacks werden für die Base-coat-Lackierung mit 17,31 g Al-Stammlösung (8 %ig) bestehend aus

| | |
|---|---|
| 12,65 g | ®SILBERLINE SS 3334AR, 60 %ig (Silberline Ltd.) |
| 56,33 g | CAB Lösung (Zusammensetzung wie oben) |
| 20,81 g | Polyesterharz ®DYNAPOL H700 |
| 2,60 g | Melaminharz ®MAPRENAL MF650 |
| 7,59 g | ®SOLVESSO 150 |

gemischt und auf ein Aluminiumblech spritzappliziert (Nassfilm ca. 20 µm). Nach einer Abdunstzeit von 30 Minuten bei Raumtemperatur wird ein TSA-Lack bestehend aus

| | |
|---|---|
| 29,60 g | Acrylharz ®URACRON 2263 XB, 50 %ig in Xylol/Butanol (Chem. Fabrik Schweizerhalle), |
| 5,80 g | Melaminharz ®CYMEL 327, 90 %ig in Isobutanol, |
| 2,75 g | Butylglycolacetat, |
| 5,70 g | Xylol, |
| 1,65 g | n-Butanol |

| | |
|---|---|
| 0,50 g | Siliconöl, 1 %ig in Xylol, |
| 3,00 g | Lichtschutzmittel ®TINUVIN 900, 10 %ig in Xylol (Ciba) |
| 1,00 g | Lichtschutzmittel ®TINUVIN 292, 10 %ig in Xylol (Ciba) |

als Top-coat-Lackierung spritzappliziert (Nassfilm ca. 50 µm). Anschliessend wird der Lack nach weiteren 30 Minuten Abdunsten bei Raumtemperatur, 30 Minuten bei 130°C eingebrannt.

## Patentansprüche

1. Feinteilige 1,4-Diketopyrrolo-[3,4-c]-pyrrole der Formel 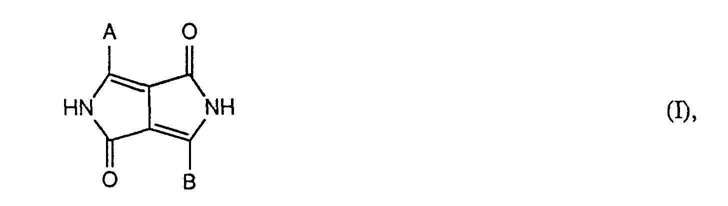 worin A und B unabhängig voneinander einen Rest der Formel 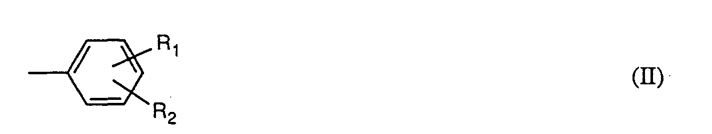 bedeuten, worin R₁ und R₂ unabhängig voneinander Wasserstoff, Halogen, C₁-C₅-Alkyl oder Phenyl sind, **dadurch gekennzeichnet sind, dass** mindestens 84 Gew.% der Teilchen einen Stokes-äquivalenten Durchmesser von ≤ 0,25 µm aufweisen, und Gemische davon.

2. 1,4-Diketopyrrolo-[3,4-c]-pyrrole der Formel I, gemäss Anspruch 1, **dadurch gekennzeichnet, dass** mindestens 84 Gew.% der Teilchen einen Stokes-äquivalenten Durchmesser von ≤ 0,20 µm aufweisen.

3. 1,4-Diketopyrrolo-[3,4-c]-pyrrole, gemäss Anspruch 2, **dadurch gekennzeichnet, dass** in Formel I A und B unabhängig voneinander Reste der Formeln 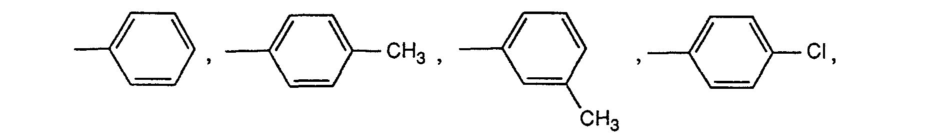 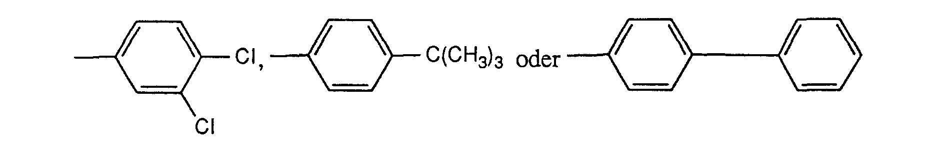 bedeuten. bedeuten.

4. 1,4-Diketopyrrolo-[3,4-c]-pyrrole, gemäss Anspruch 2, **dadurch gekennzeichnet, dass** in Formel I A und B gleich sind und Reste der Formeln 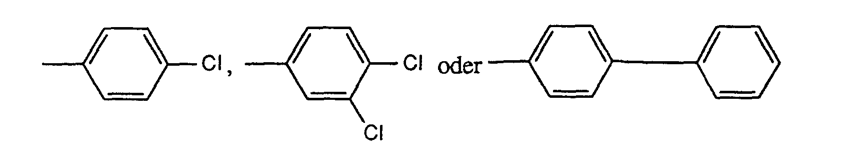 bedeuten.

5. 1,4-Diketopyrrolo-[3,4-c]-pyrrole gemäss Anspruch 2, **dadurch gekennzeichnet, dass** es sich um eine feste Lösung aus den Verbindungen der Formeln 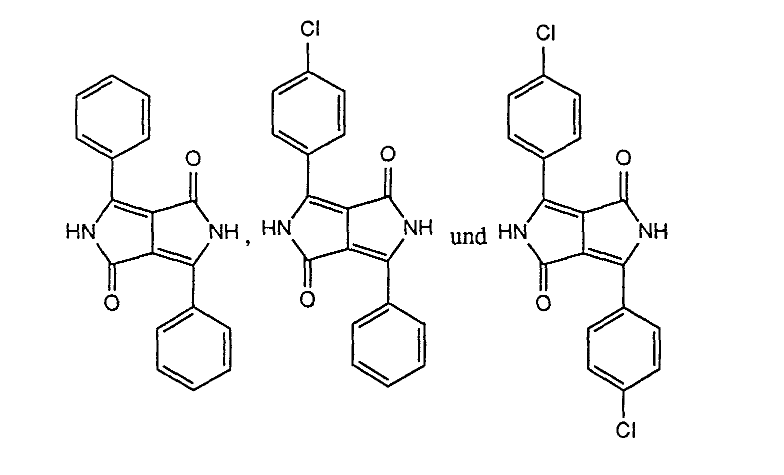 handelt.

6. 1,4-Diketopyrrolo-[3,4-c]-pyrrol der Formel 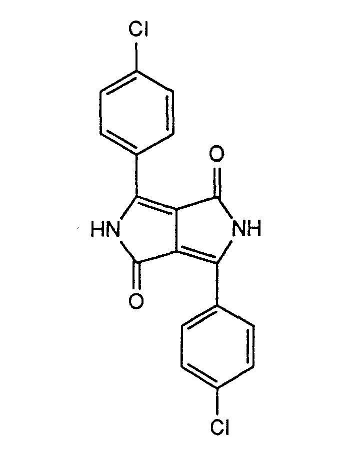 gemäss Anspruch 2, **dadurch gekennzeichnet, dass** es in einer gemäss DIN 53775, Teile 2 und 7, hergestellten Vollton-PVC-P-Pressfolie mit 1 % Pigmentkonzentration von 1,0 mm Dicke ein Chroma C*_{ab} ≥ 42 im CIELAB-System aufweist.

7. 1,4-Diketopyrrolo-[3,4-c]-pyrrol der Formel 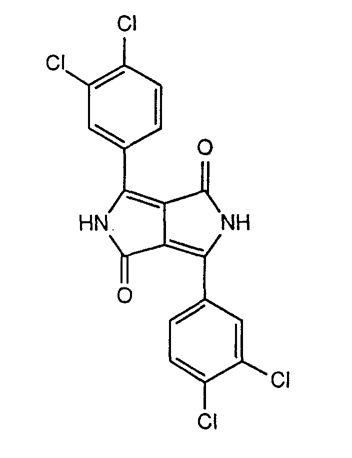 gemäss Anspruch 2, **dadurch gekennzeichnet, dass** es in einer gemäss DIN 53775, Teile 2 und 7, hergestellten Vollton-PVC-P-Pressfolie mit 1 % Pigmentkonzentration von 1,0 mm Dicke ein Chroma C*_{ab} ≥ 42 im CIELAB-System aufweist.

8. 1,4-Diketopyrrolo-[3,4-c]-pyrrol der Formel 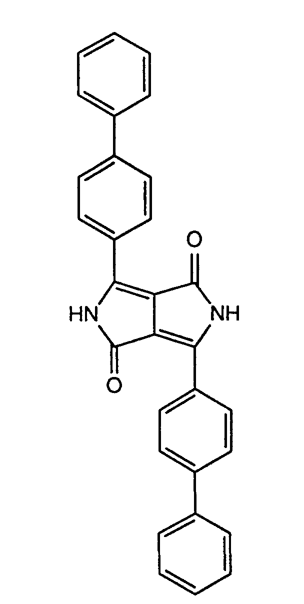 gemäss Anspruch 2, **dadurch gekennzeichnet, dass** es in einer gemäss DIN 53775, Teile 2 und 7, hergestellten Vollton-PVC-P-Pressfolie mit 1 % Pigmentkonzentration von 1,0 mm Dicke ein Chroma C*_{ab} ≥ 14 im CIELAB-System aufweist.

9. Die feste Lösung aus den 1,4-Diketopyrrolo-[3,4-c]-pyrrolen gemäss Anspruch 5, **dadurch gekennzeichnet, dass** es in einer gemäss DIN 53775, Teile 2 und 7, hergestellten Vollton-PVC-P-Pressfolie mit 1 % Pigmentkonzentration von 1,0 mm Dicke ein Chroma C*_{ab} ≥ 36 im CIELAB-System aufweist.

10. Verfahren zur Herstellung von 1,4-Diketopyrrolo-[3,4-c]-pyrrolen der Formel I gemäss Anspruch 1, durch Umsetzung von 1 Mol eines Bernsteinsäure-dicyclohexylesters, -dialkylesters, -monoalkylmonophenylesters oder -diphenylesters, wobei im Bernsteinsäureesterrest Alkyl C₁-C₁₈-Alkyl und Phenyl unsubstituiertes oder durch ein oder zwei Halogenatome, eine oder zwei C₁-C₆-Alkyl- oder C₁-C₆-Alkoxygruppen substituiertes Phenyl bedeuten, mit 2 Mol eines Gemisches von Nitrilen der Formeln
A-CN (III) und B-CN (IV),
worin A und B die oben angegebene Bedeutung haben, wobei im Nitrilgemisch ACN und BCN im Molverhältnis 100:0 bis 50:50 zueinander stehen, in einem inerten organischen Lösungsmittel in Gegenwart eines Alkalimetalls oder eines Alkalialkoholates als starke Base bei erhöhter Temperatur zu einem Pigmentalkalimetallsalz und anschliessende Freisetzung einer Verbindung der Formel I durch Protonierung des entstandenen Pigmentalkalimetallsalzes und nachfolgende Konditionierung, **dadurch gekennzeichnet, dass** die Pigmentalkalisalz-Suspension in Wasser und/oder einen Alkohol ROH, worin R C₁-C₄-Alkyl ist, bei einer Temperatur zwischen -20 und 50°C in Gegenwart einer Säure ausgetragen wird und 10 Minuten bis 48 Stunden ebenfalls bei einer Temperatur zwischen -20°C und 50°C behandelt wird, mit der Massgabe, dass die Hydrolyse nicht sequentiell in mindestens zwei Schritten ausgeführt wird.

11. Verfahren gemäss Anspruch 10, **dadurch gekennzeichnet, dass** die Säure vor oder zusammen mit der Pigmentalkalisalz-Suspension zum Wasser und/oder Alkohol zugegeben wird.

12. Verfahren gemäss Anspruch 10, **dadurch gekennzeichnet, dass** die Reste A und B unabhängig voneinander 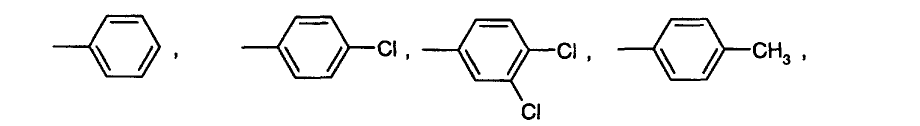 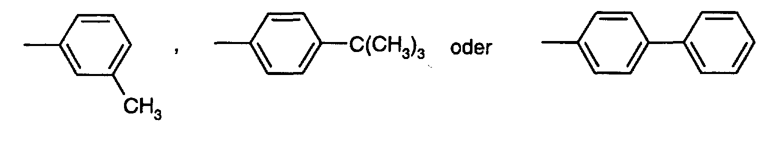 bedeuten.

13. Verfahren gemäss Anspruch 10, **dadurch gekennzeichnet, dass** die Reste A und B gleich sind.

14. Verfahren gemäss Anspruch 10, **dadurch gekennzeichnet, dass** ein Gemisch aus Wasser und Alkohol im Verhältnis 80-20:20-80 Vol% eingesetzt wird.

15. Verfahren gemäss Anspruch 10, **dadurch gekennzeichnet, dass** der Alkohol Methanol oder Ethanol ist.

16. Verfahren gemäss Anspruch 10, **dadurch gekennzeichnet, dass** die Säure in genügender Menge, um den pH < 10 am Ende der Protonierung zu erreichen, eingesetzt wird.

17. Verfahren gemäss Anspruch 10, **dadurch gekennzeichnet, dass** zur Protonierung Salzsäure, Schwefelsäure oder Phosphorsäure verwendet wird.

18. Verfahren gemäss Anspruch 10, **dadurch gekennzeichnet, dass** zur Protonierung Ameisensäure, Essigsäure, Propionsäure, Buttersäure, Hexansäure, Oxalsäure, Benzoesäure, Phenylessigsäure, Benzolsulfonsäure oder p-Toluolsulfonsäure verwendet wird.

19. Verfahren gemäss Anspruch 17, **dadurch gekennzeichnet, dass** Schwefelsäure verwendet wird.

20. Verfahren gemäss Anspruch 18, **dadurch gekennzeichnet, dass** Essigsäure oder Ameisensäure verwendet werden.

21. Verfahren gemäss Anspruch 10, **dadurch gekennzeichnet, dass** das Protonierungsmittel in beliebigen Mischungsverhältnissen zwischen 5 und 20 Gew.-Teilen des Protonierungsmittels auf 1 Teil des Pigmentalkalimetallsalzes eingesetzt wird.

22. Verfahren gemäss Anspruch 10, **dadurch gekennzeichnet, dass** Protonierung und Konditionierung bei einer Temperatur zwischen -10° und 30°C erfolgen.

23. Verwendung der 1,4-Diketopyrrolo-[3,4-c]-pyrrole gemäss Anspruch 1 als Pigmente zum Einfärben von hochmolekularem organischem Material.

## Claims

1. Finely divided 1,4-diketopyrrolo[3,4-c]pyrroles of the formula 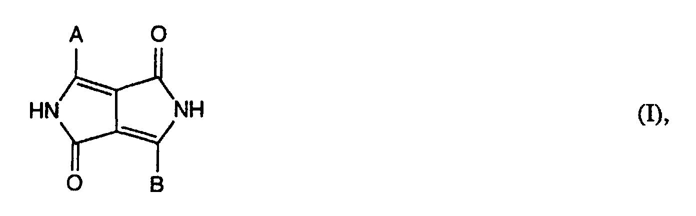 where A and B are each independently of the other a radical of the formula 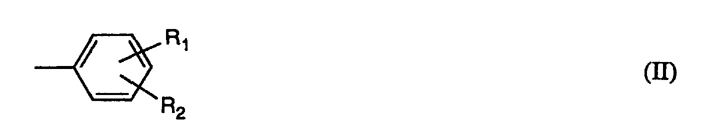 where R₁ and R₂ are each independently of the other hydrogen, halogen, C₁-C₅alkyl or phenyl, wherein at least 84 % by weight of the particles have a Stokes equivalent diameter of s 0.25 µm, and mixtures thereof.

2. 1,4-Diketopyrrolo[3,4-c]pyrroles of the formula I according to claim 1, wherein at least 84 % by weight of the particles have a Stokes equivalent diameter of ≤ 0.20 µm.

3. 1,4-Diketopyrrolo[3,4-c]pyrroles according to claim 2, wherein, in the formula I, A and B are independently of each other radicals of the formulae

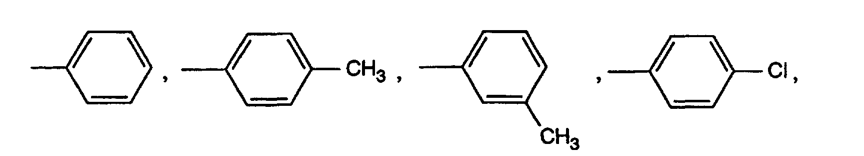

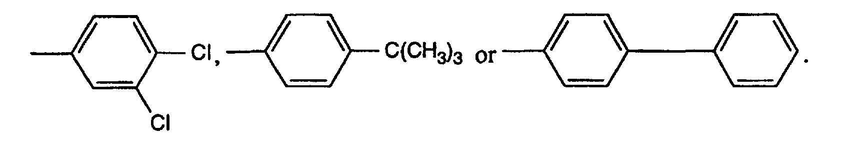

4. 1,4-Diketopyrrolo[3,4-c]pyrroles according to claim 2, wherein, in the formula I, A and B are identical and are radicals of the formulae

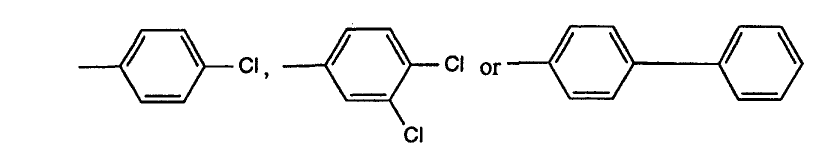

5. 1,4-Diketopyrrolo[3,4-c]pyrroles according to claim 2, comprising a solid solution of the compounds of the formulae

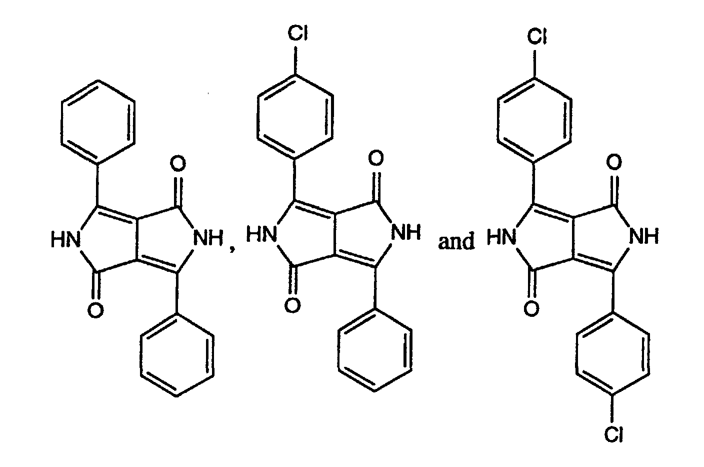

6. A 1,4-diketopyrrolo[3,4-c]pyrrole of the formula 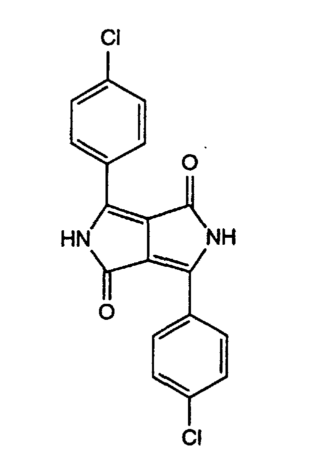 according to claim 2, having a CIELAB chroma C*_{ab} ≥ 42 in 1 % concentration in a 1.0 mm thick compression-moulded masstone PVC-P sheet produced according to DIN 53775 Parts 2 and 7.

7. A 1,4-diketopyrrolo[3,4-c]pyrrole of the formula 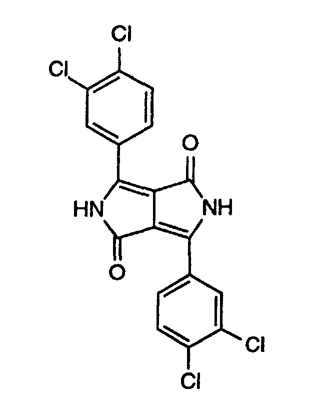 according to claim 2, having a CIELAB chroma C*_{ab} ≥ 42 in 1 % concentration in a 1.0 mm thick compression-moulded masstone PVC-P sheet produced according to DIN 53775 Parts 2 and 7.

8. A 1,4-diketopyrrolo[3,4-c]pyrrole of the formula 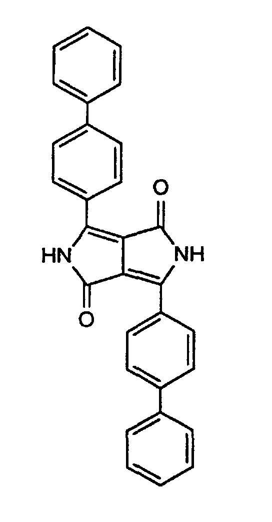 according to claim 2, having a CIELAB chroma C*_{ab} ≥ 14 in 1 % concentration in a 1.0 mm thick compression-moulded masstone PVC-P sheet produced according to DIN 53775 Parts 2 and 7.

9. The solid solution of the 1,4-diketopyrrolo[3,4-c]pyrroles according to claim 5, having a CIELAB chroma C*_{ab} ≥ 36 in 1 % concentration in a 1.0 mm thick compression-moulded masstone PVC-P sheet produced according to DIN 53775 Parts 2 and 7.

10. A process for preparing 1,4-diketopyrrolo[3,4-c]pyrroles of the formula I according to claim 1 by reacting 1 mol of a dicyclohexyl, dialkyl, alkyl phenyl or diphenyl succinate in which alkyl is C₁-C₁₈alkyl and phenyl is unsubstituted phenyl or phenyl substituted by one or two halogen atoms or one or two C₁-C₆alkyl or C₁-C₆alkoxy groups with 2 mol of a mixture of nitriles of the formulae
A-CN (III) and B-CN (IV),
where A and B are each as defined above and ACN and BCN in the nitrile mixture are in a molar ratio of 100:0 to 50:50 relative to each other, in an inert organic solvent in the presence of an alkali metal or an alkali metal alkoxide as strong base at elevated temperature to form a pigment alkali metal salt and then liberating a compound of the formula I by protonating the resulting pigment alkali metal salt and subsequent conditioning, which comprises discharging the pigment alkali metal salt suspension into water and/or an alcohol ROH, where R is C₁-C₄alkyl, at a temperature between -20 and 50°C in the presence of an acid and treating it likewise at a temperature between -20°C and 50°C for 10 minutes to 48 hours, with the proviso, that the hydrolysis is not effected sequential in at least two steps.

11. A process according to claim 10, wherein the acid is added to the water and/or alcohol before or together with the alkali metal pigment salt suspension.

12. A process according to claim 10, wherein the radicals A and B are each independently of the other

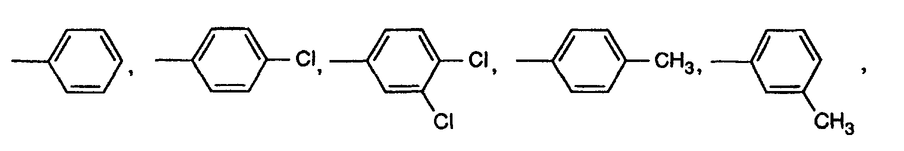

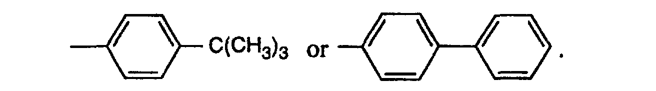

13. A process according to claim 10, wherein the radicals A and B are identical.

14. A process according to claim 10, wherein a mixture of water and alcohol in a ratio of 80-20:20-80 % by volume is used.

15. A process according to claim 10, wherein the alcohol is methanol or ethanol.

16. A process according to claim 10, wherein the acid is used in a sufficient amount to obtain pH < 10 at the end of the protonation.

17. A process according to claim 10, wherein the protonation is effected using hydrochloric acid, sulfuric acid or phosphoric acid.

18. A process according to claim 10, wherein the protonation is effected using formic acid, acetic acid, propionic acid, butyric acid, hexanoic acid, oxalic acid, benzoic acid, phenylacetic acid, benzenesulfonic acid or p-toluenesulfonic acid.

19. A process according to claim 17, wherein sulfuric acid is used.

20. A process according to claim 18, wherein acetic acid or formic acid are used.

21. A process according to claim 10, wherein the protonating agent is used in any desired mixing ratios between 5 and 20 parts by weight of the protonating agent per 1 part of the alkali metal pigment salt.

22. A process according to claim 10, wherein protonation and conditioning are carried out at a temperature between -10 and 30°C.

23. The use of the 1,4-diketopyrrolo[3,4-c]pyrroles of claim 1 as pigments for colouring macromolecular organic material.

## Revendications

1. 1,4-Dicétopyrrolopyrrolo-[3,4-c]-pyrroles finement divisés de formule 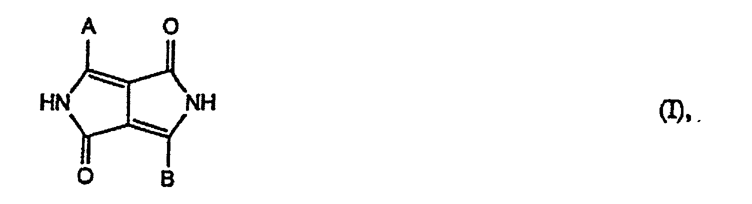 où A et B représentent, indépendamment l'un de l'autre, un reste de formule 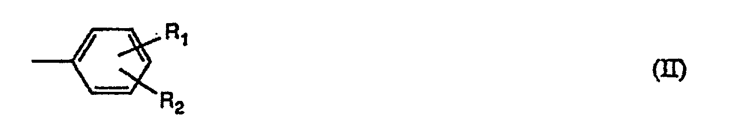 où R₁ et R₂ représentent, indépendamment l'un de l'autre, un atome d'hydrogène, un atome d'halogène, un groupe alkyle en C₁-C₅ ou phényle, **caractérisés en ce que** au moins 84 % des particules présentent un diamètre équivalent de Stokes de ≤0,25 µm, et leurs mélanges.

2. 1,4-Dicétopyrrolopyrrolo-[3,4-c]-pyrroles de formule I selon la revendication 1, **caractérisés en ce que** au moins 84 % des particules présentent un diamètre équivalent de Stokes de ≤ 0,20 µm.

3. 1,4-Dicétopyrrolopyrrolo-[3,4-c]-pyrroles selon la revendication 2, **caractérisés en ce que** à la formule I A et B représentent, indépendamment l'un de l'autre, des restes de formules

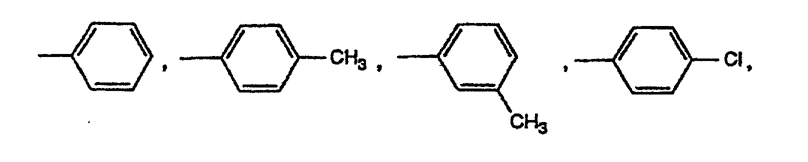

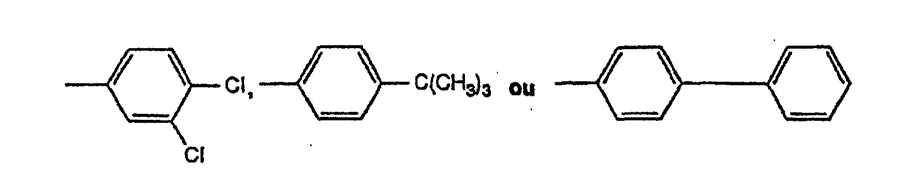

4. 1,4-Dicétopyrrolopyrrolo-[3,4-c]-pyrroles selon la revendication 2, **caractérisés en ce que** à la formule I A et B sont identiques et représentent des restes de formules

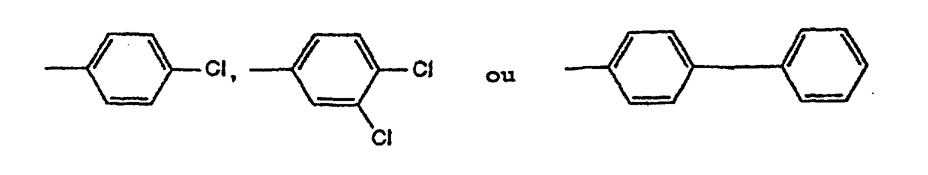

5. 1,4-Dicétopyrrolopyrrolo-[3,4-c]-pyrroles selon la revendication 2, **caractérisés en ce qu'**il s'agit d'une solution solide des composés de formules

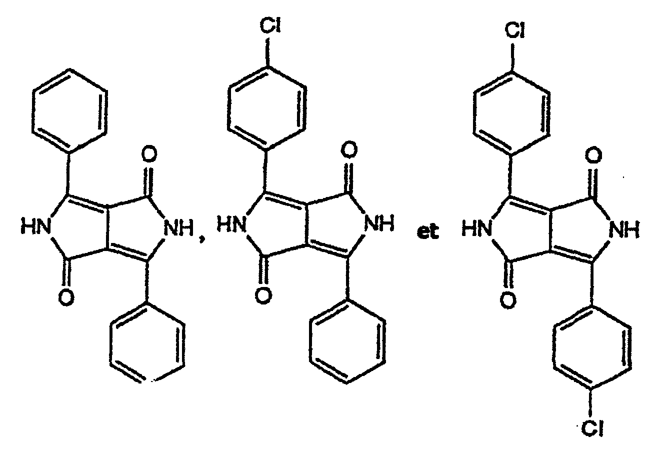

6. 1,4-Dicétopyrrolopyrrolo-[3,4-c]-pyrrole de formule 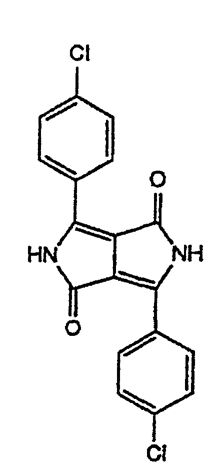 selon la revendication 2, **caractérisé en ce qu'**il présente dans une feuille pressée de PVC-P de ton plein d'une épaisseur de 1,0 mm, préparée selon la norme DIN 53775 parties 2 et 7, présentant une concentration de 1 % en pigment, une chromie C*_{AB} ≥42 dans le système CIELAB.

7. 1,4-Dicétopyrrolo-[3,4-c]pyrrole de formule 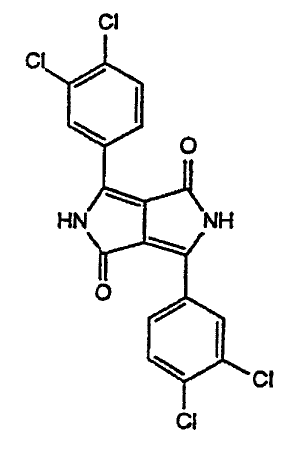 selon la revendication 2, **caractérisé en ce qu'**il présente dans une feuille pressée de PVC-P de ton plein d'une épaisseur de 1,0 mm, préparée selon la norme DIN 53775 parties 2 et 7, présentant une concentration de 1 % en pigment, une chromie C*_{AB} ≥42 dans le système CIELAB.

8. 1,4-Dicétopyrrolo-[3,4-c]pyrrole de formule 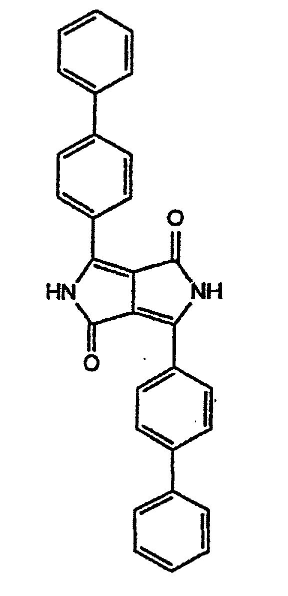 selon la revendication 2, **caractérisé en ce qu'**il présente dans une feuille pressée de PVC-P de ton plein d'une épaisseur de 1,0 mm, préparée selon la norme DIN 53775 parties 2 et 7, présentant une concentration de 1 % en pigment, une chromie C*_{AB} ≥14 dans le système CIELAB.

9. Solution solide de 1,4-dicétopyrrolo-[3,4-c]pyrroles selon la revendication 5, **caractérisée en ce qu'**elle présente dans une feuille pressée de PVC-P de ton plein d'une épaisseur de 1,0 mm, préparée selon la norme DIN 53775 parties 2 et 7, présentant une concentration de 1 % en pigment, une chromie C*_{AB} ≥36 dans le système CIELAB.

10. Procédé pour la préparation de 1,4-dicétopyrrolo-[3,4-c]-pyrroles de formule I selon la revendication 1, par réaction de 1 mole d'un ester dicyclohexylique, d'un ester dialkylique, d'un ester monoalkyl-monophénylique ou d'un ester diphénylique de l'acide succinique, dans le reste d'acide succinique le groupe alkyle représente alkyle en C₁-C₁₈ et le groupe phényle représente phényle non substitué ou substitué par un ou deux atomes d'halogène, un ou deux groupes alkyle en C₁-C₆ ou alkoxy en C₁-C₆, sur 2 moles d'un mélange de nitriles de formules
A-CN (III) et B-CN (IV),
où A et B possèdent la signification donnée ci-dessus, ACN et BCN sont présents dans le mélange de nitriles dans un rapport molaire de 100:0 à 50:50, dans un solvant organique inerte en présence d'un métal alcalin ou d'un alcoolate alcalin en tant que base forte, à température élevée, pour obtenir un sel de métal alcalin de pigment et par libération ultérieure d'un composé de formule I par protonation du sel de métal alcalin de pigment formé et par conditionnement ultérieur, **caractérisé en ce qu'**on extrait la suspension de sel alcalin de pigment dans l'eau et/ou un alcool ROH, où R représente un groupe alkyle en C₁-C₄, à une température comprise entre -20 et 50°C en présence d'un acide, et on effectue un traitement pendant une durée de 10 minutes à 48 heures à une température de comprise également entre -20 et 50°C, à condition que l'hydrolyse soit mise en oeuvre en au moins deux étapes de façon non séquentielle.

11. Procédé selon la revendication 10, **caractérisé en ce qu'**on ajoute l'acide à l'eau et/ou alcool avant ou conjointement avec la suspension de sel alcalin de pigment.

12. Procédé selon la revendication 10, **caractérisé en ce que** les restes A et B représentent indépendamment l'un de l'autre

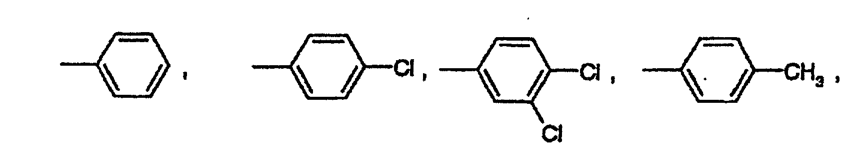

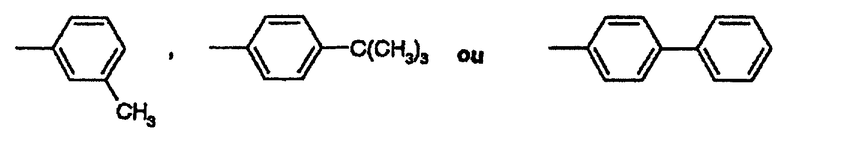

13. Procédé selon la revendication 10, **caractérisé en ce que** les restes A et B sont identiques.

14. Procédé selon la revendication 10, **caractérisé en ce qu'**on utilise un mélange d'eau et d'alcool dans un rapport 80-20:20-80 % en volume.

15. Procédé selon la revendication 10, **caractérisé en ce que** l'alcool est le méthanol ou l'éthanol.

16. Procédé selon la revendication 10, **caractérisé en ce qu'**on utilise l'acide dans une quantité suffisante, afin d'atteindre le pH <10 à la fin de la protonation.

17. Procédé selon la revendication 10, **caractérisé en ce qu'**on utilise pour la protonation l'acide chlorhydrique, l'acide sulfurique ou l'acide phosphorique.

18. Procédé selon la revendication 10, **caractérisé en ce qu'**on utilise pour la protonation l'acide formique, l'acide acétique, l'acide propionique, l'acide butyrique, l'acide hexanoïque, l'acide oxalique, l'acide benzoïque, l'acide phénylacétique, l'acide benzènesulfonique ou l'acide p-toluènesulfonique.

19. Procédé selon la revendication 17, **caractérisé en ce qu'**on utilise l'acide sulfurique.

20. Procédé selon la revendication 18, **caractérisé en ce qu'**on utilise l'acide acétique ou l'acide formique.

21. Procédé selon la revendication 10, **caractérisé en ce qu'**on utilise l'agent de protonation dans des rapports de mélange quelconques compris entre 5 et 20 parties en poids de l'agent de protonation pour 1 partie du sel de métal alcalin de pigment.

22. Procédé selon la revendication 10, **caractérisé en ce que** la protonation et le conditionnement sont mis en oeuvre à une température comprise entre -10° et 30°C.

23. Utilisation des 1,4-dicétopyrrolo-[3,4-c]pyrroles selon la revendication 1 en tant que pigments pour la teinture de matière organique de haut poids moléculaire.
